# EUROPEAN PATENT APPLICATION

(11) **EP 4 119 659 A1**
(43) Date of publication of application: **18.01.2023**
(21) Application number: 21186225.5
(22) Date of filing: 16.07.2021
(51) Int. Cl.: C12N 9/16, A23K 10/16

(54) **PHYTASE VARIANTS**

(71) Applicant: AB Enzymes Oy, 05200 Rajamäki (FI)
(72) Inventor: JUNTUNEN, Kari, 05200 Rajamäki (FI); AHOLA, Pihla, 05200 Rajamäki (FI); BENSON, Sven, 70569 Stuttgart (DE); LORENZ, Lenz, 70569 Stuttgart (DE); METZGER, Tara, 64293 Darmstadt (DE); KÜHN, Imke, 64293 Darmstadt (DE); PURANEN, Terhi, 05200 Rajamäki (FI); PALOHEIMO, Marja, 05200 Rajamäki (FI)
(74) Representative: Espatent Oy

(57) **Abstract**

A phytase variant, a method for its manufacturing, an animal feed, a feed supplement, a dry and a liquid formulation, a method of degrading phytic acid, a method of manufacturing a phytase variant, a method of manufacturing a feed pellet, and a recombinant host cell configured to produce at least one polypeptide are disclosed, wherein the phytase variant comprises amino acid substitutions and has at least 95% amino acid sequence identity with amino acids of SEQ ID NO: 1.

## Description

### TECHNICAL FIELD

The present disclosure generally relates to field of enzyme technology. The disclosure relates particularly, though not exclusively, to phytase variants having an improved thermostability. The present phytase variants are useful in various applications where degradation of phytate is desired, such as in feedstuffs.

### BACKGROUND

This section illustrates useful background information without admission of any technique described herein representative of the state of the art.

Phytic acid (phytate, inositol hexakisphosphate, IP6) is found in many plants where it functions as storage of phosphate. Phosphate stored in IP6 molecules can be released as inorganic phosphate. When inorganic phosphate is released from phytic acid molecules, IP6 is converted to lower inositol phosphates pentaphosphate (IP5), tetraphosphate (IP4), trisphosphate (IP3), bisphosphate (IP2) and monophosphate (IP1).

Phytases are a group of phosphatase enzymes that catalyse the hydrolysis of phytic acid. Commercially available phytases belong to the histidine acid phosphatase (HAP) protein family. The phytases belonging to the HAP protein family share conserved N-terminal active site heptapeptide motif RHGXRXP and the catalytically active HD-dipeptide at the C-terminus. Histidine acid phosphatases are part of a larger superfamily of histidine phosphatases. They share a conserved catalytic core centred on a histidine, which becomes phosphorylated during the course of the reaction. PFAM motif His_Phos_2 (PF00328) represents branch 2 of the histidine phosphatase superfamily, the branch containing mainly acid phosphatases and phytases.

Phytases are used in feeds to improve phosphate availability from feed ingredients of plant origin (e.g. wheat, barley, corn, soybean) by phytate degradation. This is in particular of interest for monogastric animals like poultry, fish and pigs, because intestinal phytate degradation in the upper intestinal tract of these animals is limited. This limitation not only restricts utilisation of phosphorus, but also the availability of other nutrients due to the chelating effect of inositol phosphates. Consequently, IP6 should be dephosphorylated as far as possible, preferably to IP1 to further increase availability of phosphate and other nutrients.

It is an object of the present disclosure to provide phytase variants that exhibit phytase activity and that have thermostability that allows their use in industrial processes. Another object of the present disclosure is to provide phytase variants with improved properties when used in industrial processes. Yet another object of the present disclosure is to provide phytase variants that can be used in enzyme compositions for phytate degradation.

### SUMMARY

The appended independent claims define the scope of protection. Any example or technical description of a product and/or method in the description and/or drawings not covered by the claims is presented not as an embodiment of the invention, but as background art or as an example which is useful for understanding the claimed invention.

According to a first aspect is provided a phytase variant having at least 80% sequence identity with SEQ ID NO: 1, and an amino acid substitution in at least one position selected from the positions 33, 45, 54, 89, 101, 139, 166, 175, 176, 179, 184, 185, 202, 262, 280, 340, 393, 397, 398, and 412, wherein the phytase variant has an improved thermostability compared to the SEQ ID NO: 1.

Advantageously, the present phytase variant has improved thermostability compared to the parent phytase having the SEQ ID NO: 1. Improved thermostability of the present phytase variant means that the variant remains functionally stable (for example retains its enzyme activity) and/or chemically stable (for example does not unfold or denature) after being exposed to high temperatures as compared to the parent phytase. The improved thermostability can be used to an advantage when high level of recovery of the phytase is needed after demanding process steps such as exposures to high temperature where phytase activity may be lost. Further, the improved thermostability is advantageous when a manufacturing process of a product containing the present phytase variant comprises further steps where high temperature is involved in feed processing like conditioning, extrusion, and pelleting. As shown in the examples provided below, the claimed phytase variant has a thermostability that is improved compared to the parent phytase enzyme, allowing the present variant to withstand demanding processing conditions and/or have stability against proteases.

In an embodiment the present phytase variant has at least IP6 degrading activity.

In an embodiment is provided a functional fragment of the present phytase variant, the functional fragment having phytase activity.

In an embodiment the amino acid substitution in the phytase variant is selected from N33, Q45, E54, A89, A101, K139, A166, A175, A176, Y179, Q184, T185, L202, A262, S280, N340, S393, L397, Q398, and S412.

In an embodiment the amino acid substitution is selected from X33C, X45P, X54C/A, X89T, X101C, X139L/C, X166R, X175T, X176K, X179C, X184R, X185Q, X202C, X262N, X280M, X340R, X393P, X397F, X398R, and X412Q; preferably from N33C, Q45P, E54C/A, A89T, A101C, K139L/C, A166R, A175T, A176K, Y179C, Q184R, T185Q, L202C, A262N, S280M, N340R, S393P, L397F, Q398R, and S412Q.

In an embodiment the phytase variant has at least two amino acid substitutions in the amino acids X33C and X179C, or X54C and X101C, or X139C and X202C; or X175T and X184R, or X280M and X397F, or X280M and X398R; or in the amino acids N33C and Y179C, or E54C and A101C, or K139C and L202C, or A175T and Q184R, or S280M and L397F, or S280M and Q398R. The two amino acid substitutions may have a stabilizing effect on the phytase variant when present together in the phytase variant. For example additional or alternative disulfide bridges may form with substitutions that change an amino acid to a cysteine residue.

In an embodiment the phytase variant comprises at least two amino acid substitutions in the amino acids X33C and X179C.

In an embodiment the phytase variant comprises at least two amino acid substitutions in the amino acids X54C and X101C.

In an embodiment the phytase variant comprises at least two amino acid substitutions in the amino acids X139C and X202C.

In an embodiment the phytase variant comprises at least two amino acid substitutions in the amino acids X175T and X184R.

In an embodiment the phytase variant comprises at least two amino acid substitutions in the amino acids X280M and X397F.

In an embodiment the phytase variant comprises at least two amino acid substitutions in the amino acids X280M and X398R.

In an embodiment the phytase variant comprises at least two amino acid substitutions in the amino acids in the amino acids N33C and Y179C.

In an embodiment the phytase variant comprises at least two amino acid substitutions in the amino acids E54C and A101C.

In an embodiment the phytase variant comprises at least two amino acid substitutions in the amino acids K139C and L202C.

In an embodiment the phytase variant comprises at least two amino acid substitutions in the amino acids A175T and Q184R.

In an embodiment the phytase variant comprises at least two amino acid substitutions in the amino acids S280M and L397F.

In an embodiment the phytase variant comprises at least two amino acid substitutions in the amino acids S280M and Q398R.

In an embodiment the phytase variant further comprises at least one additional amino acid substitution in at least one position selected from the positions 45, 89, 166, 175, 176, 184, 185, 280, 340, 393, 397, 398, and 412; the substitution being preferably X45P, X89T, X166R, X175T, X176K, X184R, X185Q, X280M, X340R, X393P, X397F, X398R, or X412Q; and more preferably the substitution is Q45P, A89T, A166R, A175T, A176K, Q184R, T185Q, S280M, N340R, S393P, L397F, Q398R, or S412Q.

According to a second aspect is provided a method of preparing the present phytase variant by recombinantly expressing the phytase variant in a host cell.

According to a third aspect is provided composition comprising the present phytase variant and at least one additive selected from a stabilizer, a preservative, a mineral, and a nutrient.

According to a third aspect is provided an animal feed comprising the present phytase variant or the present composition, and at least one protein source of plant or animal origin, and/or an energy source of plant or animal origin, and:
a. Optionally at least one further enzyme selected from protease, amylase, phytase, xylanase, endoglucanase, beta-glucanase, pectinase, cellulase, hemicellulase, mannanase, phosphatase, galactosidase, lipase, pectinase, pentosanase, or a combination thereof; and
b. Optionally at least one filler selected from maltodextrin, flour, salt, sodium chloride, sulfate, sodium sulfate, or a combination thereof.

According to a fourth aspect is provided a feed supplement comprising the present phytase variant or the present composition; and:
a. Optionally at least one further enzyme selected from protease, amylase, phytase, xylanase, endoglucanase, beta-glucanase, pectinase, cellulase, hemicellulase, mannanase, phosphatase, galactosidase, lipase, pectinase, pentosanase, or a combination thereof; and
b. Optionally at least one carrier or ingredient selected from maltodextrin, flour, salt, sodium chloride, sulfate, sodium sulfate, minerals, amino acids, prebiotics, probiotics, vitamins, or a combination thereof.

According to a fifth aspect is provided a liquid formulation comprising the present phytase variant or the present composition, and water.

According to a sixth aspect is provided a dry formulation comprising the present phytase variant or the present composition.

According to a seventh aspect is provide a method of degrading phytic acid and/or phytate comprising contacting the present phytase variant or the present composition with the phytic acid and/or phytate.

In an embodiment the phytic acid and/or phytate are provided in a feed ingredient.

According to an eighth aspect is provided a method of manufacturing a phytase variant in a recombinant host cell comprising:
a. providing a polynucleotide comprising genetic elements configured to produce the present phytase variant; and
b. expressing the polynucleotide in a recombinant host cell;
wherein the phytase variant is capable of releasing phosphate from phytic acid

In an embodiment the method further comprises selecting at least one substitution position such that the unfolding temperature of the phytase variant increases at least 0.2°C in the same conditions compared to a phytase having the amino acid sequence of SEQ ID NO: 1, and using a polynucleotide encoding such phytase variant in step a. In another embodiment the substitution position is selected such that the unfolding temperature increases at least 0.1, 0.2, 0.3, 0.4, 0.5, 0.6, 0.7, 0.8, 0.9 or 1°C. In another embodiment the substitution position is selected such that the unfolding temperature increases at least 1, 5, 10 or 15°C. The at least one substitution position can be selected from a position identified in Table 1 either alone or together with one more position identified for any BA59 variant in Table 1.

A method of manufacturing a feed pellet or a feed extrude comprising extruding a composition comprising the present phytase variant.

A recombinant host cell comprising genetic elements configured to produce at least one polypeptide having an amino acid sequence having at least 80% sequence identity with SEQ ID NO: 1.

In an embodiment the recombinant host cell is selected from the group consisting of plant cells, fungal cells, filamentous fungal cells, yeasts, and bacterial cells, preferably from filamentous fungal cells, most preferably from *Trichoderma,* even more preferably from *Trichoderma reesei.*

### BRIEF DESCRIPTION OF THE FIGURES

Some example embodiments will be described with reference to the accompanying figures, in which:

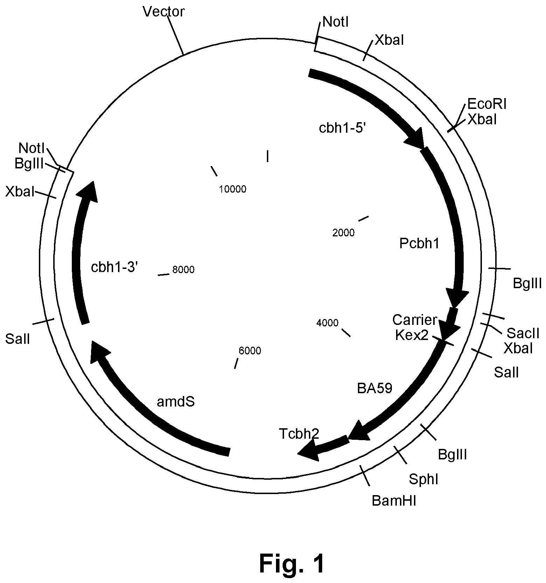

**Figure 1** shows a schematic picture of the expression plasmid used in the transformation of *Trichoderma reesei* for expression of the BA59 phytase. Similar constructions were used for expression of the phytase variant genes. The expression of the recombinant genes in the host cell was controlled by use of the following genetic elements: *T. reesei cbh1* promoter (Pcbh1) for transcription initiation, and *T. reesei cbh2* (Tcbh2) terminator for transcription termination. *T. reesei cbh2* carrier encoding the CBHII CBM and linker region (Carrier) was used instead of the native phytase signal sequence and Kex2 protease cleavage site (Kex2) was included between the encoded carrier polypeptide and phytase. A synthetic gene (amdS) encoding the AmdS marker was included for selection of the transformants and *T. reesei cbh1* 3'- and 5'-flanking regions (cbh1-5' and cbh1-3', respectively) were used to optionally target the expression cassette to *cbh1* locus. The vector backbone (Vector) derives from pUC19. Picture was generated using Clone Manager Professional 9 from Sci-Ed Software. A selection of restriction enzyme sites is shown in the picture.

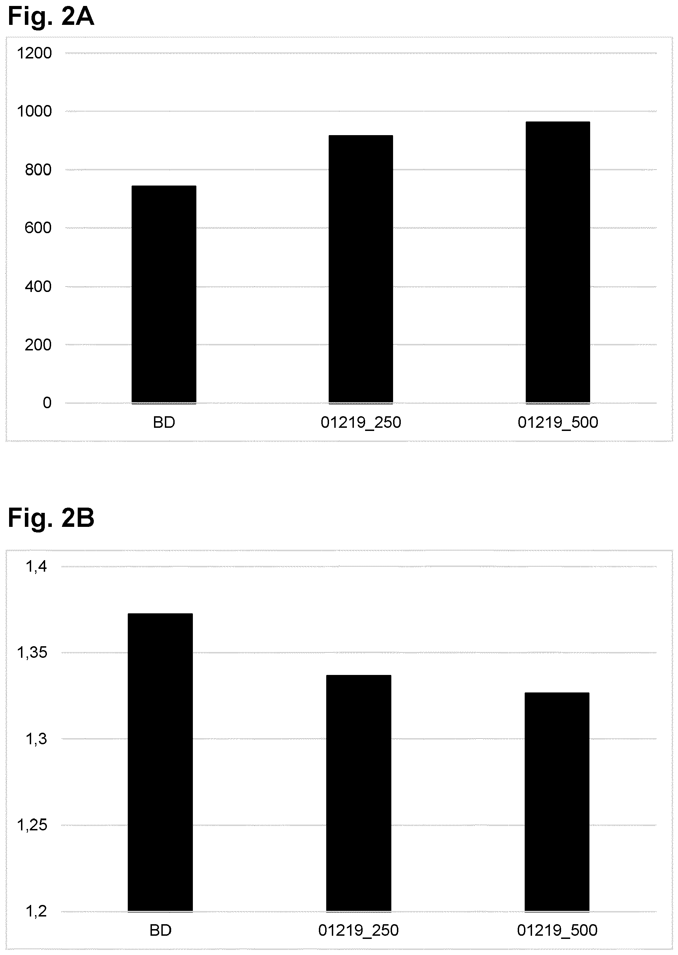

**Figure 2** outlines the feeding trial performance evaluation. In the broiler trial birds were fed with a basal diet that was reduced in phosphorus with the phytase variant 01219 (CND-PHY-01219) and performance in birds was compared to birds fed with the same diet without phytase addition. Data obtained from the trial is shown in Fig. 2A and B. Broiler performance was improved by both phytase dosages of variant 01219 tested demonstrating the efficiency of the phytases to release phytate bound phosphorus in broilers.
**Fig. 2A****:** Body weight of 21 days (d) old broilers in grams (g). BD = basal diet reduced in P and Ca. Phytase fed bird received BD diets added with 250 or 500 FTU/kg phytase.
**Fig. 2B****:** Feed conversion ratio (FCR) of 21 days (d) old broilers in grams (g) / g. BD = basal diet reduced in P and Ca. Phytase fed bird received BD diets added with 250 or 500 FTU/kg phytase.

**SEQUENCE LISTING**

| | |
|---|---|
| SEQ ID NO: 1 | Amino acid sequence of mature parent BA59 phytase without native signal peptide. |

### DETAILED DESCRIPTION

The present invention also relates to a polynucleotide comprising a nucleic acid sequence encoding a phytase variant according to the present invention.

As used herein, the term "phytase" means an enzyme having capability to enzymatically degrade phytic acid to lower inositol phosphates.

In an embodiment the polypeptide comprising the phytase variant comprises at least one further amino acid sequence selected from a signal sequence, a secretory sequence, a carrier polypeptide, a binding domain, a tag, enzyme, a linker, or any combination thereof.

As used herein, the term "variant" means a sequence or a fragment of a sequence (nucleotide or amino acid) inserted, substituted or deleted by one or more nucleotides/amino acids, or which is chemically modified. The term variant may in some embodiments also include a sequence of the phytase variant, or a fusion protein including the phytase variant.

The terms "phytase variant" and "variant of phytase" mean a phytase molecule obtained by site-directed or random mutagenesis, insertion, substitution, deletion, recombination and/or any other protein engineering method, and which leads into a genetically modified phytase that differs in its amino acid sequence from the parent phytase. The terms "wild type phytase ", "wild type enzyme", "wild type", or "wt" in accordance with the disclosure, describe a phytase enzyme with an amino acid sequence found in nature or a fragment thereof. The variant encoding gene can be synthesised, or the parent gene be modified using genetic methods, e.g., by site-directed mutagenesis, a technique in which one or more than one mutation is introduced at one or more defined sites in a polynucleotide encoding the parent polypeptide. The term variant phytase may also be referred to by using a name given to the variant in the examples, e.g., BA59.

As used herein, the term "mature polypeptide" means any polypeptide wherein at least one signal sequence or signal peptide or signal peptide and a putative pro-peptide is cleaved off. For example, the "mature polypeptide" of BA59 comprises the amino acids of SEQ ID NO: 1.

As used herein, a "peptide" and a "polypeptide" are amino acid sequences including a plurality of consecutive polymerized amino acid residues. For purpose of this disclosure, peptides are molecules including up to 20 amino acid residues, and polypeptides include more than 20 amino acid residues. The peptide or polypeptide may include modified amino acid residues, naturally occurring amino acid residues not encoded by a codon, and non-naturally occurring amino acid residues. As used herein, a "protein" may refer to a peptide or a polypeptide of any size. A protein may be an enzyme, a protein, an antibody, a membrane protein, a peptide hormone, regulator, or any other protein.

As used herein, "sequence identity" means the percentage of exact matches of amino acid residues between two optimally aligned sequences over the number of positions where there are residues present in both sequences. When one sequence has a residue with no corresponding residue in the other sequence, the alignment program allows a gap in the alignment, and that position is not counted in the denominator of the identity calculation.

As used herein, "sequence alignment" of the amino acid sequences means, aligning the sequences using Clustal Omega (1.2.4) multiple sequence alignment program (https://www.ebi.ac.uk/Tools/msa/clustalo/) as described by Sievers et al 2011, and using the default settings.

Unless otherwise specified, all references to a certain amino acid position refer to an amino acid of the SEQ ID NO: 1 in said position, or to an amino acid present or missing in the corresponding position of an amino acid sequence aligned with SEQ ID NO: 1.

As used herein, the term "disulfide bridge", or "disulfide bond", or "SS bridge" refers to a bond formed between the sulfur atoms of cysteine residues in a polypeptide or a protein. Disulfide bridges can be naturally occurring, or non-naturally occurring, and, for example, introduced by way of amino acid substitution(s).

As used herein, the term "corresponding positions" or "corresponding amino acid position" means aligning at least two amino acid sequences according to identified regions of similarity or identity as pairwise alignment or as multiple sequence alignment, thereby pairing up the corresponding amino acids.

As used herein, "amino acid substitution" means an amino acid residue replacement with an amino acid residue that is different than the original amino acid in that specific replacement position. The term "amino acid substitution" can refer to conservative amino acid substitutions and non-conservative amino acid substitutions, which means the amino acid residue is replaced with an amino acid residue having a similar side chain (conservative), or a different side chain (non-conservative), as the original amino acid residue in that place.

The term "functional fragment" means a fragment or portion of the current variant, which retains about the same enzymatic function or effect.

The term "secretory signal sequence" or "signal sequence" or a "secretory peptide" refers to an amino acid sequence which is a component or a part of a larger polypeptide, and which directs the larger polypeptide through a secretory pathway of a host cell in which it is produced. The secretory signal sequence can be native or it can be obtained from another source. Depending on the host cell, the larger polypeptide may be cleaved from the secretory peptide during transit through the secretory pathway, thereby forming a mature polypeptide lacking the secretory peptide.

"Phytase activity" as used herein, refers to the phytic acid degrading activity. Example 3 provide examples of a method for determining phytase activity.

In an embodiment the term "enzyme composition" means an enzymatic fermentation product, possibly isolated and purified, from a single species of a microorganism. The enzyme composition usually comprises a number of different enzymatic activities produced by the microorganism. In another embodiment the enzyme composition is a mixture of monocomponent enzymes, preferably enzymes derived from bacterial or fungal species by using conventional recombinant production techniques.

As used herein, a "host cell" means any cell type that is susceptible to transformation, transfection, transduction, mating, crossing, CRISPR-Cas, or the like with a nucleic acid construct or expression vector comprising a polynucleotide. The term "host cell" encompasses any progeny that is not identical due to mutations that occur during replication. Non-limiting examples of a host cell are fungal cells, preferably a filamentous fungal cell (e.g., *Trichoderma* or *Trichoderma reesei, Aspergillus* or *Aspergillus oryzae* or *Aspergillus niger, Myceliophthora* or *Myceliophthora thermophila, Thermothelomyces or Thermothelomyces heterothallica* or *Humicola or Humicola insolens* or *Fusarium* or *Fusarium venenatum),* bacterial cells, preferably gram-positive Bacilli (e.g., *Bacillus subtilis, B. licheniformis, B. megaterium, B. amyloliquefaciens, B. pumilus),* gram-negative bacteria (e.g., *Escherichia coli),* actinomycetales (e.g., *Streptomyces sp., Nonomuraea flexuosa)* and yeasts (e.g., *Saccharomyces cerevisiae, Schizosaccharomyces pombe, Pichia pastoris, Yarrowia lipolytica).*

In another embodiment the phytase variant is obtained by recombinant production in plant cells, i.e., in a transgenic plant.

The recombinant host cell can be used to produce the phytase variant and to contain a polynucleotide encoding it. The recombinant phytase variant can be operably linked to one or more control sequences that direct the production of the variant. The recombinant host cell is useful also in preparation of variants with different properties. For example, a host cell can be selected, which provides post-translational modifications beneficial for stability or activity, or which facilitates post-processing of a variant produced in the host cell.

In an embodiment the host cell is non-pathogenic. This is particularly advantageous for using the host cell or the phytase variant produced in it for food or animal feed.

In an embodiment the composition containing the phytase variant is food or feed, and it may further comprise plant material which contains phytic acid.

In an embodiment the composition is a food additive or a feed additive further comprising at least one of: at least one trace mineral, at least one amino acid, in particular lysine, water soluble vitamin, fat soluble vitamin, prebiotic, and probiotic.

In an embodiment the composition is a food additive or a feed additive complying with the requirements of Regulation (EC) No 1831/2003 of the European Parliament and of the Council of 22 September 2003 on additives for use in animal nutrition.

The term "hemicellulase" as used herein refers to a group of enzymes capable of catalyzing hydrolysis of hemicellulosic material. Hemicellulases include, for example, arabinases, arabinofuranosidases, certain acetylmannan esterases, acetylxylan esterases, ferulyoyl esterases, mannanases, mannosidases, xylanases, and xylosidases.

In an embodiment the composition is in a form of a liquid composition or a solid composition such as solution, dispersion, paste, pellet, powder, granule, coated granule, tablet, cake, crystal, crystal slurry, gel, extrude, precipitate, premix, or a combination thereof.

The term "stability" refers to the stability of the phytase variant as a function of time in a certain environmental condition. Different methods are used to analyze the stability of phytase variants. The unfolding temperature of a phytase variant can be measured to assess the thermostability of the phytase variant, as described in the Example 4. The stability and residual activity can be measured by using the "activity assay" with adjusted parameters as described in the Examples. The term "stability" includes stability during use in a process with high temperature conditions and/or during storage and/or stability against proteases.

The term "promoter" refers to a portion of a gene containing DNA sequence(s) that provide for the binding of RNA polymerase and initiation of transcription. Promoter sequences are commonly, but not always, found in the 5' non-coding regions of genes.

The term "propeptide" or "pro-peptide" is a part of a protein that is cleaved during maturation or activation. Once cleaved, a propeptide generally has no independent biological function.

As used herein, the terms "domain" and "region" can be used interchangeably with the term "module".

The term "catalytic domain" or "catalytic module" or "core" denotes a domain of an enzyme, which may or may not have been modified or altered, but which has retained at least part of its original activity.

The following abbreviations are used for amino acids:

| | | |
|---|---|---|
| A | Ala | Alanine |
| C | Cys | Cysteine |
| D | Asp | Aspartic acid |
| E | Glu | Glutamic acid |
| F | Phe | Phenylalanine |
| G | Gly | Glycine |
| H | His | Histidine |
| I | Ile | Isoleucine |
| K | Lys | Lysine |
| L | Leu | Leucine |
| M | Met | Methionine |
| N | Asn | Asparagine |
| P | Pro | Proline |
| Q | Gln | Glutamine |
| R | Arg | Arginine |
| S | Ser | Serine |
| T | Thr | Threonine |
| V | Val | Valine |
| W | Trp | Tryptophan |
| Y | Tyr | Tyrosine |
| X | Xaa | Any one of the above amino acids |

Substitutions are described herein by using of the following nomenclature: amino acid residue in the protein scaffold; position; substituted amino acid residue(s). According to this nomenclature the substitution of, for instance, a single residue of alanine to tyrosine residue at position 23 is indicated as Ala23Tyr or A23Y. A substitution of any amino acid in position 23 to tyrosine is indicated as Xaa23Tyr or X23Y or 23Y. A substitution of a tyrosine in position 179 to phenylalanine, tryptophan or leucine is indicated as Y179F/W/L.

As used herein, the term "comprising" includes the broader meanings of "including", "containing", and "comprehending", as well as the narrower expressions "consisting of" and "consisting only of".

As used herein, the term "expression" includes any step or all steps involved in the production of a polypeptide in a host cell including, but not limited to, transcription, translation, post-translational modification, and secretion. Expression may be followed by harvesting, i.e. recovering, the host cells or the expressed product.

In an embodiment the phytase variant has phytase activity, and an amino acid sequence with at least 80%, at least 85%, at least 90%, at least 95 %, at least 96 %, at least 97 %, at least 98 %, or at least 99% sequence identity with amino acids 1 - 414 of SEQ ID NO: 1. In an embodiment the variant polypeptide does not have 100% sequence identity with amino acids 1 - 414 of SEQ ID NO: 1. In an embodiment, the amino acid numbering of the variant polypeptide corresponds to that of SEQ ID NO: 1. In an alternative embodiment, the amino acid numbering of the variant polypeptide corresponds to that of SEQ ID NO: 1 partially.

In an embodiment the phytase variant comprises at least one, two, or three additional disulfide bridges that are not naturally present in a phytase having the SEQ ID NO: 1.

In an embodiment the phytase variant comprises one, two or three disulfide bridge between residues X33C and X179C, X54C and X101C, and X139C and X202C.

In an embodiment the phytase variant comprises one, two or three disulfide bridge between residues N33C and Y179C, E54C and A101C, and K139C and L202C.

In an embodiment the phytase variant comprises at least one substitution selected from N33E, Q45K, K139M/V/L, A176N, Y179F/W/L, Q184C, Q185R, L202M, A262E, and S393R.

In an embodiment the phytase variant does not contain at least one substitution selected from N33E, Q45K, K139M/V/L, A176N, Y179F/W/L, Q184C, Q185R, L202M, A262E, S393R, or any combination thereof.

In an embodiment the substitutions to obtain additional cysteine residues for disulfide bridging are made by genetic engineering, and the disulfide bridge thereby obtained is artificial, i.e..not present in the parent phytase. In an embodiment the cysteine residues are obtained through genetic modification of the parent gene. e.g. with site directed mutagenesis, the disulfide bridge thereby obtained between the said cysteine residues being also non-naturally occurring. Many protein engineering approaches or alternatively gene synthesis can be used to create amino acid substitutions at a specific location in an amino acid chain, allowing the natural or wild-type amino acid at a specific location to be substituted with another amino acid residue. In an embodiment at least two amino acid residues in the variant polypeptide amino acid region are substituted with cysteines, thereby enabling a disulfide bond formation between the sulfur atoms of the substituent cysteine residues.

In an embodiment, the at least one additional disulfide bridge stabilizes the phytase variant, thereby increasing its thermostability compared to a variant or parent phytase without said additional disulfide bridge.

In an embodiment the phytase variant comprises the substitutions X45P, X89T, X139L, X166R, X176K, X185Q, X262N, X340R, X393P, X398R, and X412Q; or Q45P, A89T, K139L, A166R, A176K, T185Q, A262N, N340R, S393P, Q398R, and S412Q.

In an embodiment the total number of the amino acid substitutions in the phytase variant, compared to the SEQ ID NO: 1, is 1-15, 1-12, 1-10, 1-5 or 1-3.

In an embodiment the variant polypeptide, or the functional fragment, has a predicted molecular weight between 40 and 55 kDa, preferably between 45-50 kDa. The predicted molecular weight can be determined by calculating the sum of the molecular weights of the individual amino acids in the variant polypeptide, or in its functional fragment.

In an embodiment the variant polypeptide has an improved stability and/or activity compared to the polypeptide having the sequence SEQ ID NO: 1.

Providing phytase variants that retain stability in high temperatures is advantageous for applications wherein the phytase need to survive exposure to high temperature. High temperature tolerance of phytase variants may have other benefits like increased specific activity, enzyme storage stability or improved stability against proteases.

In an embodiment the composition is provided in the form of a liquid composition or a solid composition, such as solution, dispersion, paste, powder, granule, granulate, coated granulate, tablet, cake, crystal, crystal slurry, gel, or pellet.

In an embodiment the phytic acid is degraded in a plant-based material or partly plant based material which contains phytic acid.

In an embodiment the present phytase variant is used in a animal feed, and the animal is a ruminant or a non-ruminant. In another embodiment the animal is a cattle like beef or cow, a sheep or goat. In another embodiment the non-ruminant include poultry (such as broiler, layer and turkey and duck); pigs (such as piglets, growing pigs and sows); fish (such as salmonids, carp, tilapia and catfish) and crustaceans.

In an embodiment the feed is animal feed intended to be fed to animals such as any compound feed or mixture. In another embodiment feed comprises or consists of grains such as maize, wheat, oats, barley, sorghum and rice; protein sources like soybean meal, sunflower meal and canola meal as well as of minerals. The feed, wherein the present variant is used, has improved nutritional value compared to a feed without the variant. The present composition and the present phytase variant degrade phytic acid of the feed and thereby increase its nutritional value for the animals. The animal feed, wherein the present phytase variant or the present composition is used, can be formulated in the form of a wet composition or a dry composition.

### EXAMPLES

The following examples are provided to illustrate various aspects of the present invention. They are not intended to limit the invention, which is defined by the accompanying claims.

### Example 1. Structural modelling of 6-phytase sequences and design of thermostable Budvicia aquatica BA59 phytase variants

The crystal structure of *E. coli* 6-phytase was obtained from the PDB database and used as structural model for computational analyses. For SEQ ID NO. 1 a structural model for computational analyses was generated by comparative modelling using phytase from *Yersinia kristensenii* (PDB: 4ARV) as template.

All atoms not part of the protein chain in the structural model of 6-phytase were removed and hydrogens were added according to a pH of 5.0. A rectangular simulation box was added with a minimal distance of 10 Angstroms to the protein atoms and subsequently filled with water molecules and ions to yield a solvated and neutralized system. To obtain an equilibrated system at room temperature, atom velocities were assigned from a Boltzmann distribution according to 300 K and the system was equilibrated coupled to a heat bath and a barostat at a temperature of 300 K and a pressure of 1.0 bar (system EQ300). To obtain an additional system equilibrated at a high temperature, the pressure coupling was removed, new atom velocities were drawn from a Boltzmann distribution according to 400 K and the system was equilibrated coupled to a heat bath at a temperature of 400 K (system EQ400).

For the system equilibrated at 300 K (EQ300) as well as for the system equilibrated at 400 K (EQ400), multiple production simulation runs with newly assigned particle velocities drawn from a Boltzmann distribution according to the respective temperatures were conducted. For both systems, the individual trajectories of the production runs were combined and subsequently analyzed. The root mean square fluctuation (RMSF) was calculated for all Ca atoms over all frames of the combined trajectory at 300K (RMSFCa-300K) as well as for all Ca atoms over all frames of the combined trajectory at 400K (RMSFCa-400K). The difference of the RMSF at both temperatures was calculated as ΔRMSFCα = RMSFCα-400K - RMSFCα-300K. To obtain representative conformations of the production simulations, both combined trajectories were clustered by the Ca root mean square deviation (RMSD). Mean Ca RMSD values (RMSDCa-mean) were then calculated between all conformations of the largest clusters of the combined trajectory at 400 K and all conformations of the largest clusters of the combined trajectory at 300 K for all Ca atoms. A set of temperature sensitive positions with either a high ΔRMSFCα or a high RMSDCa-mean value was derived by assessment of the analysis results. All temperature sensitive positions as well as positions in close proximity to temperature sensitive positions were subsequently analyzed by geometrical criteria to derive candidates for amino acid substitutions with a potentially stabilizing effect (substitution candidates). For each of the thus derived substitution candidates, a system was constructed, equilibrated, simulated and analyzed as described above to yield ΔRMSFCα and RMSDCa-mean values. Substitution candidates were ranked by their improvement in conformational stability according to ΔRMSFCα and RMSDCa-mean values and the most promising candidates were selected for experimental characterization.

The designed *Budvicia aquatica* BA59 phytase variants are described in detail in Table 1.

**Table 1. List of BA59 variants designed. The amino acid numbering corresponds to the amino acid numbering of the mature parent BA59 molecule presented in SEQ ID NO: 1. The present phytase variant may have one or more of the mutation(s) specified below, or a completed set of mutation(s) specified below for any variant.**

| **Variant code** | **Mutation(s)** |
|---|---|
| PA31 | A166R |
| PA34 | A175T |
| PA38 | T185Q |
| PA46 | S280M |
| PA50 | N340R |
| PA62 | S393P |
| PA64 | L397F |
| PA65 | Q398R |
| PA70 | S412Q |
| PA98 | Q184R |
| CND-PHY-00048 | S280M, L397F |
| CND-PHY-00049 | S280M, Q398R |
| CND-PHY-00053 | A175T, Q184R |
| CND-PHY-00079 | S393P, Q398R |
| CND-PHY-00081 | N340R, S393P |
| CND-PHY-00083 | A166R, T185Q |
| CND-PHY-00084 | A166R, A176K, T185Q |
| CND-PHY-00086 | Q45P, A89T |
| CND-PHY-00407 | Q45P, A89T, K139L, A166R, A176K, T185Q, A262N, N340R, S393P, Q398R, S412Q |
| CND-PHY-00643 | Q45P, E54C, A101C |
| CND-PHY-00674 | L397F, Q398R |
| CND-PHY-00675 | L397F, S412Q |
| CND-PHY-00676 | Q398R, S412Q |
| CND-PHY-00787 | K139C, L202C |
| CND-PHY-01152 | Q45P, E54A, A89T, K139C, A166R, A176K, T185Q, L202C, N340R, S393P, Q398R, S412Q |
| CND-PHY-01156 | N33C, Q45P, A89T, K139C, A166R, A176K, Y179C, T185Q, L202C, N340R, S393P, Q398R, S412Q |
| CND-PHY-01160 | Q45P, E54C, A89T, A101C, K139C, A166R, A176K, T185Q, L202C, N340R, S393P, Q398R, S412Q |
| CND-PHY-01162 | Q45P, A89T, K139C, A166R, A175T, A176K, Q184R, T185Q, L202C, N340R, S393P, Q398R, S412Q |
| CND-PHY-01163 | Q45P, A89T, K139C, A166R, A176K, T185Q, L202C, S280M, N340R, S393P, L397F, Q398R, S412Q |
| CND-PHY-01197 | N33C, Q45P, E54A, A89T, K139C, A166R, A176K, Y179C, T185Q, L202C, N340R, S393P, Q398R, S412Q |
| CND-PHY-01200 | Q45P, E54A, A89T, K139C, A166R, A175T, A176K, Q184R, T185Q, L202C, N340R, S393P, Q398R, S412Q |
| CND-PHY-01201 | Q45P, E54A, A89T, K139C, A166R, A176K, T185Q, L202C, S280M, N340R, S393P, L397F, Q398R, S412Q |
| CND-PHY-01211 | N33C, Q45P, A89T, K139C, A166R, A175T, A176K, Y179C, Q184R, T185Q, L202C, N340R, S393P, Q398R, S412Q |
| CND-PHY-01212 | N33C, Q45P, A89T, K139C, A166R, A176K, Y179C, T185Q, L202C, S280M, N340R, S393P, L397F, Q398R, S412Q |
| CND-PHY-01218 | Q45P, E54C, A89T, A101C, K139C, A166R, A175T, A176K, Q184R, T185Q, L202C, N340R, S393P, Q398R, S412Q |
| CND-PHY-01219 | Q45P, E54C, A89T, A101C, K139C, A166R, A176K, T185Q, L202C, S280M, N340R, S393P, L397F, Q398R, S412Q |
| CND-PHY-01222 | Q45P, A89T, K139C, A166R, A175T, A176K, Q184R, T185Q, L202C, S280M, N340R, S393P, L397F, Q398R, S412Q |
| CND-PHY-01327 | E54C, A101C |
| CND-PHY-01328 | E54C, A101C, K139C, L202C |
| CND-PHY-01330 | Q45P, A89T, K139C, A166R, A176K, T185Q, L202C, S280M, N340R, S393P, L397F, S412Q |
| CND-PHY-01332 | Q45P, E54C, A89T, A101C, K139C, A166R, A176K, T185Q, L202C, S280M, N340R, S393P, L397F, S412Q |

### Example 2. Production of phytase variants

Standard molecular biology methods were used in the isolation and enzyme treatments of DNA (e.g., isolation of plasmid DNA, digestion of DNA to produce DNA fragments), in *E. coli* transformations, sequencing etc. The basic laboratory methods used were either as described by the enzyme, reagent or kit manufacturer, or as described in the standard molecular biology handbooks, e.g., Sambrook and Russell (2001), or as described in the following examples.

The phytase genes encoding the designed *Budvicia aquatica* BA59 phytase variants (Table 1) were ordered as synthetic genes using codons optimised for expression in *Trichoderma reesei.* The codon optimised gene encoding the mature BA59 phytase (SEQ ID NO:2) was used as backbone for the synthetic genes encoding the BA59 variant phytases.

The phytases in the constructions were expressed from *T. reesei cbh1* (*cel7A*) promoter using a carrier polypeptide (CBM and linker) encoding sequence from *T. reesei cbh2* (*cel6A*). A Kex2 protease cleavage site was included between the carrier polypeptide and phytase like described in Paloheimo et al. 2003. The transcription was terminated using *cbh2* terminator, followed in the construction by a synthetic amdS marker gene. In addition, the constructions contain *cbh1* 3' and 5' flanking regions for optionally targeting the expression vector into the *cbh1* locus. A schematic picture of an expression plasmid is shown in Figure 1.

Circular expression plasmids were transformed in *T. reesei* protoplasts. The transformants were selected on plates containing acetamide as the sole nitrogen source. The host strain used lacks the four major endogenous *T. reesei* cellulases: CBHI/Cel7A, CBHII/Cel6A, EGl/Cel7B and EGII/Cel5A. The transformations were performed according to Penttilä et al, 1987, with the modifications described in Karhunen et al., 1993. Alternatively, CRISPR-Cas technology can be used in transformations.

The transformants were sporulated on potato dextrose agar (PDA) prior to cultivation.

The transformants were cultivated on 96-well plates (Havukainen et al, 2020) to analyse the phytase production of the transformants. Phytase activity of the recombinant BA59 and variant phytases was measured from the culture supernatants as release of inorganic phosphate from sodium phytate. Production of the recombinant protein was also detected from the culture supernatant by sodium dodecyl sulphate polyacrylamide gel electrophoresis (SDS-PAGE). The unfolding temperatures of phytases were analysed using Prometheus NT.48 equipment (NanoTemper GmbH, Munich, Germany).

All the transformants produced phytase activity. Transformants producing the most thermostable BA59 variant phytases and the reference strain producing the parent BA59 phytase were purified on selection plates through single conidia prior to sporulating them on PDA. The purified, selected transformants were cultivated in shake flasks and/or bioreactors in complex cellulase-inducing medium to obtain material for further characterisation and application tests.

### Example 3. Phytase activity assays

Phytase activity was analysed using sodium phytate (C₆H₆Na₁₂O₂₄P₆, LabChem EE05501) as a substrate in a concentration of 12.7 mM.

The activity of samples from the microtiter plate cultivations (Example 2) was screened using Fluent^{®} automation workstation (Tecan Group Ltd, Männedorf, Switzerland) as follows. The samples used in the assay were diluted in a 0.2 M citrate buffer (pH 5.0) containing 0.01 % Tween 20 (Merck 822184). 0.01 % of Tween 20 was added also to the substrate solution. 200 µl of sample dilution and 200 µl of substrate were mixed and incubated at 37 °C. After exactly 15 min incubation 400 µl of 15 % (w/v) TCA solution (Trichloroacetic acid, CCl₃COOH, Merck 807) was added to the mixture to stop the reaction. 25 µl of reaction mixture was transferred into another well and 225 µl of water was added to make 1:10 dilution. 250 µl of colour reagent consisting of three volumes of 1M sulphuric acid (H₂SO₄, Merck 731), one volume of 2.5 % (w/v) ammonium molybdate ((NH₄)₆MO₇O₂₄ · 4 H₂O, Merck 1182) and one volume of 10 % (w/v) ascorbic acid (C₆H₈O₆, AnalaR Normapur 20150) were added and the colour reaction was incubated for 20 min at 50°C. After the incubation the absorption was measured at 820 nm. The absorbance of the sample was compared to that of a reference sample, parent BA59.

The activity from the shake flask and fermentation cultivations was analysed using a phytase activity assay (PPU). In PPU analysis one activity unit is the quantity of enzyme that liberates 1 µmol of inorganic phosphate per one minute from sodium phytate at pH 5.0 and at 37 °C in a 15 min reaction time.

The samples used in the assay were diluted in a reaction buffer (0.2 M citrate buffer, pH 5.0) and 1 ml of enzyme solution was used in the analysis. 1 ml of substrate was added to the enzyme solution and after incubating the mixture at 37 °C for exactly 15 min, the reaction was stopped by adding 2 ml of 15 % (w/v) TCA solution (Trichloroacetic acid, CCl₃COOH, Merck 807). The reaction mixture was cooled to room temperature and after this 1:10 dilution was done by mixing 0.2 ml of the mixture and 1.8 ml of water in a test tube. 2.0 ml of freshly made colour reagent was added to the tube and mixed. The colour reagent consisted of three volumes of 1 M sulphuric acid (H₂SO₄, Merck 731), one volume of 2.5 % (w/v) ammonium molybdate ((NH₄)₆MO₇O₂₄ · 4 H₂O, Merck 1182) and one volume of 10 % (w/v) ascorbic acid (C₆H₈O₆, AnalaR Normapur 20150). The tubes were incubated at 50 °C for 20 min and cooled to room temperature. After this the absorption was measured at 820 nm against the enzyme blank. For the enzyme blank the substrate was added after the TCA and the 15 min incubation was passed. The amount of liberated phosphate was determined via a standard curve of the color reaction with a phosphate solution of known concentration.

All variants disclosed in Table 1 were shown to have phytase activity.

### Example 4. Analysis of unfolding temperatures of phytases

The unfolding temperatures of phytase variants (Examples 1 and 2) were analysed from culture supernatants and/or from purified phytases using Prometheus NT.48 equipment (NanoTemper GmbH, Munich, Germany).

Prior to the measurements the samples were centrifuged for 10 min at 16000 g at 4 °C to remove any large aggregates. Standard-grade glass capillaries (NanoTemper GmbH) were filled with 10-15 µl of the sample, excitation light was preadjusted to get adequate fluorescence signals for F330 and F350, and samples were measured in temperature range 20-110°C with temperature slope of 1°C/min.

The inflection point of the unfolding curve, also known as the melting temperature (Tm), was determined from the experimental derivative or curve fitting. Protein samples with higher Tm tend to be more stable.

The parent BA59 was used as a reference in the analysis.

The unfolding temperatures of the best variants was improved up to 14.3 - 14.6 °C. The results from Prometheus analysis of selected variants are shown in Table 2.

**Table 2. Unfolding temperatures of BA59 and selected BA59 variants. The unfolding temperatures were measured using Prometheus equipment NT.48.**

| **Variant code** | **Unfolding** |
|---|---|
| BA59 | 63.0 |
| PA31 | 64.2 |
| PA38 | 63.7 |
| PA65 | 63.6 |
| PA70 | 63.2 |
| CND-PHY-00079 | 63.2 |
| CND-PHY-00081 | 63.2 |
| CND-PHY-00083 | 64.8 |
| CND-PHY-00084 | 64.8 |
| CND-PHY-00086 | 66.4 |
| CND-PHY-00407 | 66.0 |
| CND-PHY-00643 | 69.8 |
| CND-PHY-00676 | 63.0 |
| CND-PHY-00787 | 69.4 |
| CND-PHY-01152 | 73.2 |
| CND-PHY-01156 | 77.6 |
| CND-PHY-01160 | 77.3 |
| CND-PHY-01162 | 70.2 |
| CND-PHY-01163 | 72.6 |
| CND-PHY-01197 | 71.7 |
| CND-PHY-01200 | 72.1 |
| CND-PHY-01201 | 74.1 |
| CND-PHY-01211 | 74.7 |
| CND-PHY-01212 | 77.4 |
| CND-PHY-01218 | 74.7 |
| CND-PHY-01219 | 77.5 |
| CND-PHY-01222 | 71.1 |
| CND-PHY-01327 | 67.7 |
| CND-PHY-01328 | 72.7 |
| CND-PHY-01330 | 72.1 |
| CND-PHY-01332 | 76.4 |

### Example 5. Stability test in feed processing

For stability testing in feed processing the phytase sample was added to a wheat-soya bean meal-based diet which was conditioned for 30 seconds at temperatures ranging from 70 to 85°C followed by pelleting through a 3 mm die. The pellets were cooled and analysed for phytase activity.

For the test, a 300 kg batch of a mash feed was first produced, containing approximately 65% wheat, 28% soybean meal, 4% soya oil, 1% monocalcium phosphate, 1.4% limestone, 0.4% salt and 0.5% premix of vitamins and trace minerals. Then, an enzyme premix was produced by adding the enzyme sample to the above mash feed. The mash feed and enzyme were mixed for 10 minutes using a horizontal mixer. After sampling of the mash the meal (with enzyme) was heated to the target temperature by adjusting steam addition to the cascade mixer before passing the pellet die. For each temperature, a sample was taken 10 minutes after the target conditioning temperature (as measured in the meal just prior to pelleting) was achieved.

Samples from the pelleted feeds were taken and cooled by cold air stream. On a sample splitter feeds were homogeneously sub-sampled for analysis using the phytase in feed assay method (ISO 30024:2009; Animal feeding stuffs - Determination of phytase activity).

Phytase recovery was calculated relative to the activity in the corresponding feed mash sample prior to its processing. The thermostabilities of chosen BA59 variants with the highest unfolding temperatures were analysed. BA59 wt (SEQ ID NO: 1) was used as a reference.

The residual activities of BA59 variant phytases were more stable in common feed processing than the wild-type BA59 phytase. The improvement is of relevance for the use of such phytases in animal nutrition because conditioning followed by pelleting is a common process of feed production. Phytases to be used in feed need to overcome such processing to stay efficient in the animal. The residual activities are shown in Table 3.

**Table 3: Residual activity of BA59 phytase and BA59 phytase variants after different conditioning temperatures and feed pelleting compared to the activity analysed from mash feed (%)**

| Conditioning temperature | BA59 | CND-PHY-01212 | CND-PHY-01219 | CND-PHY-01332 |
|---|---|---|---|---|
| 70°C | 86 | 101 | 93 | 101 |
| 75°C | 54 | 90 | 100 | 96 |
| 80°C | 56 | 91 | 87 | 86 |
| 85°C | 51 | 63 | 75 | 61 |

### Example 6. Feeding trial

The thermophilic phytase variants described can be used in feeding of animals, e.g., pigs and broilers. They can be used alone or in combination with other enzymes, e.g., xylanase, β-glucanase, cellulase, and/or mannanase.

Effects of one of the recombinant phytase variants of the invention was studied on growth in broilers. Ultrafiltrate of the fermentation broth including the recombinant phytase was dried and target levels applied to a broiler diet. A control diet was fed without the phytase enzyme.

Phytase variant 01219 was tested in a broiler trial. Day old male Cobb 430 broilers were distributed to the different treatments (15 pens x 25 birds each). Phosphorus (P) and calcium (Ca) content of the corn-soybean meal diet was reduced in the basal diet (BD; starter feed: available phosphorus (Pav) content 2.1 g/kg and Ca 8.5 g/kg, respectively). Phytase variant 01219 was added to BD treatment feeds with defined activity levels of 250 or 500 FTU/kg feed. The phytase improved the body weight (Fig 2 A) compared to birds fed the unsupplemented basal diet by 23 and 30% for 250 and 500 FTU/kg, respectively. Because the feed intake of the phytase fed birds increased also the improvement on feed conversion ratio (FCR; Fig 2 B) was not as huge and the same for both dosages (3%). The performance improvement, specifically in the growth demonstrate that the 01219 phytase is efficient in animal feeding.

The foregoing description has provided by way of non-limiting examples of particular implementations and embodiments a full and informative description of the best mode presently contemplated by the inventors for carrying out the invention.

It is however clear to a person skilled in the art that the invention is not restricted to details of the embodiments presented in the foregoing, but that it can be implemented in other embodiments using equivalent means or in different combinations of embodiments without deviating from the characteristics of the invention.

Furthermore, some of the features of the afore-disclosed example embodiments may be used to obtain an advantage or a technical effect without the corresponding use of other features. As such, the foregoing description shall be considered as merely illustrative of the principles of the present invention, and not in limitation thereof. Hence, the scope of the invention is only restricted by the appended patent claims

### REFERENCES

Havukainen S, M Valkonen, K Koivuranta and CP Landowski. 2020. Studies on sugar trasporter CRT1 reveal new characteristics that are critical for cellulase induction in Trichoderma reesei. Biotechnol. Biofuels 2020 Sep 14;13:158. doi: 10.1186/s13068-020-01797-7. eCollection 2020.
Joutsjoki VV, TK Torkkeli and KMH Nevalainen. 1993. Transformation of Trichoderma reesei with the Hormoconis resinae glucoamylase P (gamP) gene: production of a heterologous glucoamylase by Trichoderma reesei. Curr. Genet. 24:223-228.
Karhunen T, A Mäntylä, KMH Nevalainen and PL Suominen. 1993. High frequency one-step gene replacement in Trichoderma reesei. I. Endoglucanase I overproduction. Mol. Gen. Genet. 241:515-522.
Paloheimo M, A Mäntylä, J Kallio, and P Suominen. 2003. High-yield production of a bacterial xylanase in the filamentous fungus Trichoderma reesei requires a carrier polypeptide with an intact domain structure. Appl. Env. Microbiol. 69:7073-7082.
Penttilä M, H Nevalainen, M Rättö, E Salminen and J Knowles. 1987. A versatile transformation system for the cellulolytic filamentous fungus Trichoderma reesei. Gene 61:155-164.
Sambrook J and DW Russell. 2001. Molecular cloning, a laboratory manual. Cold Spring Harbor Laboratory, New York, US.
Sievers F, A Wilm, D Dineen, TJ Gibson, K Karplus, W Li, R Lopez, H McWilliam, M Remmert, J Söding, JD Thompson and DG Higgins. 2011. Fast, scalable generation of high-quality protein multiple sequence alignments using Clustal Omega. Mol. Syst. Biol. 7:539.
Sommerfeld M Schollenberger, L Hemberle and M Rodehutscord 2017 Modification and application of an in vitro assay to examine inositol phosphate degradation in the digestive tract of poultry. Science of Food and Agiculture; 97 (12) p 4219-4226; doi.org/10.1002/jsfa.8297

## Claims

1. A phytase variant having at least 80% sequence identity with SEQ ID NO: 1, and an amino acid substitution in at least one position selected from the positions 33, 45, 54, 89, 101, 139, 166, 175, 176, 179, 184, 185, 202, 262, 280, 340, 393, 397, 398, and 412, wherein the phytase variant has an improved thermostability compared to the SEQ ID NO: 1.

2. The phytase variant of claim 1, wherein the amino acid substitution is selected from N33, Q45, E54, A89, A101, K139, A166, A175, A176, Y179, Q184, T185, L202, A262, S280, N340, S393, L397, Q398, and S412.

3. The phytase variant of claim 1 or 2, wherein the amino acid substitution is selected from:
X33C, X45P, X54C/A, X89T, X101C, X139L/C, X166R, X175T, X176K, X179C, X184R, X185Q, X202C, X262N, X280M, X340R, X393P, X397F, X398R, and X412Q; or
N33C, Q45P, E54C/A, A89T, A101C, K139L/C, A166R, A175T, A176K, Y179C, Q184R, T185Q, L202C, A262N, S280M, N340R, S393P, L397F, Q398R, and S412Q.

4. The phytase variant of any one of claims 1-3, wherein the phytase variant has at least two amino acid substitutions in the amino acids:
X33C and X179C, or X54C and X101C, or X139C and X202C; or X175T and X184R, or X280M and X397F, or X280M and X398R; or
N33C and Y179C, or E54C and A101C, or K139C and L202C, or A175T and Q184R, or S280M and L397F, or S280M and Q398R.

5. The phytase variant of any one of claims 1-4 further comprising at least one additional amino acid substitution in at least one position selected from the positions 45, 89, 166, 175, 176, 184, 185, 280, 340, 393, 397, 398, and 412; the substitution being preferably X45P, X89T, X166R, X175T, X176K, X184R, X185Q, X280M, X340R, X393P, X397F, X398R, or X412Q; more preferably Q45P, A89T, A166R, A175T, A176K, Q184R, T185Q, S280M, N340R, S393P, L397F, Q398R, or S412Q.

6. A method of preparing the phytase variant of any of claims 1-5 by recombinantly expressing the phytase variant in a host cell according to claim 17 or 18.

7. A composition comprising the phytase variant of claims 1-5 and at least one additive selected from a stabilizer, a preservative, a mineral, and a nutrient.

8. An animal feed comprising the phytase variant of claims 1-5 or the composition of claim 7, and at least one protein source of plant or animal origin and/or an energy source of plant or animal origin, and
a. Optionally at least one further enzyme selected from protease, amylase, phytase, xylanase, endoglucanase, beta-glucanase, pectinase, cellulase, hemicellulase, mannanase, phosphatase, galactosidase, lipase, pectinase, pentosanase, or a combination thereof; and
b. Optionally at least one carrier or ingredient selected from maltodextrin, flour, salt, sodium chloride, sulphate, sodium sulphate, or a combination thereof.

9. A feed supplement comprising the phytase variant of claims 1-5 or the composition of claim 7; and
a. Optionally at least one further enzyme selected from protease, amylase, phytase, xylanase, endoglucanase, beta-glucanase, pectinase, cellulase, hemicellulase, mannanase, phosphatase, galactosidase, lipase, pectinase, pentosanase, or a combination thereof; and
b. Optionally at least one carrier or ingredient selected from maltodextrin, flour, salt, sodium chloride, sulphate, sodium sulphate, minerals, amino acids, prebiotics, probiotics, vitamins, or a combination thereof.

10. A liquid formulation comprising the phytase variant of claims 1-5 or the composition of claim 7, and water.

11. A dry formulation comprising the phytase variant of claims 1-5 or the composition of claim 7.

12. A method of degrading phytic acid and/or phytate comprising contacting the phytase variant of any one of claims 1-5 or the composition of claim 7 with the phytic acid and/or phytate.

13. The method of claim 12 wherein the phytic acid and/or phytate are provided in a feed ingredient.

14. A method of manufacturing a phytase variant of claims 1-5 in a recombinant host cell comprising:
a. providing a polynucleotide comprising genetic elements configured to produce the phytase variant of claims 1-5; and
b. expressing the polynucleotide in a recombinant host cell;
wherein the phytase variant is capable of releasing phosphate from phytic acid.

15. The method of claim 14 further comprising selecting at least one substitution position such that the unfolding temperature of the phytase variant increases at least 0.1, 0.2, 0.3, 0.4, 0.5, 0.6, 0.7, 0.8, 0.9 or 1°C compared to a phytase having the amino acid sequence of SEQ ID NO: 1, and using a polynucleotide encoding such phytase variant in step a.

16. A method of manufacturing a feed pellet or a feed extrude comprising extruding a composition comprising the phytase variant of claims 1-5.

17. A recombinant host cell comprising genetic elements configured to produce at least one polypeptide having an amino acid sequence having at least 80% sequence identity with SEQ ID NO: 1.

18. The recombinant host cell of claim 17, wherein the recombinant host cell is selected from the group consisting of plant cells, fungal cells, filamentous fungal cells, yeasts, and bacterial cells, preferably from bacterial cells, most preferably from *Bacillus.*
